# EUROPEAN PATENT APPLICATION

(11) **EP 3 741 429 A1**
(43) Date of publication of application: **25.11.2020**
(21) Application number: 19175662.6
(22) Date of filing: 21.05.2019
(51) Int. Cl.: A61N 5/06

(54) **CONTROLLING THE GENERATION OF A LIGHT PULSE BY A DEVICE**

(71) Applicant: Koninklijke Philips N.V., 5656 AG Eindhoven (NL)
(72) Inventor: VAN KEMPEN, Eric Gerard Marie, 5656 AE Eindhoven (NL); PEETERS, Felix Godfried Peter, 5656 AE Eindhoven (NL)
(74) Representative: de Haan, Poul Erik

(57) **Abstract**

According to an aspect, there is provided an apparatus for controlling the generation of a light pulse by a device, The device is configured to apply the light pulse to skin of a subject to perform a treatment operation on or to the skin, and the apparatus comprises a processing unit configured to receive a first measurement signal from a first sensor configured to monitor at least one eye of a user of the device during use of the device; process the first measurement signal to determine an amount of closure of the at least one eye of the user; determine whether each of one or more criteria is satisfied, said one or more criteria comprising at least a closure criterion requiring the at least one eye of the user to be closed by at least a minimum amount of closure; and activate the device to generate a light pulse if it is determined that each of said one or more criteria is satisfied.

## Description

### FIELD OF THE INVENTION

This disclosure relates to the control of the generation of a light pulse by a device, and particularly to the control of the generation of a light pulse where the light pulse is applied to skin of a subject to perform a treatment operation on or to the skin.

### BACKGROUND OF THE INVENTION

Light-based hair removal is a treatment used to inhibit the growth of hair by exposing the skin to bright flashes or pulses of light, which can be referred to as IPL (Intense Pulsed Light) where the light pulse is generated by a lamp or light bulb. Alternatively the flash or pulse can be generated using a laser or one or more light emitting diodes (LEDs). The light penetrates the skin and is absorbed - among other places - in the root of the hair. The temperature of the root of the hair will rise and subsequently the temperature of the surrounding tissue will also rise. The growth of the hair is inhibited if the temperature rise is sufficient. This process is known as photothermolysis. The pulse of light therefore needs to contain a minimum amount of energy, because sufficiently high temperatures are needed to be generated at the root of the hair and its surrounding tissue for photothermolysis to occur.

It is known that a bright pulse of light can cause a so-called 'after image' in the vision of someone viewing the pulse. This effect is well known and is related to the fatigue of the photoreceptors in the eyes (i.e. temporary photo-pigment bleaching). Such an after image can be perceived as unpleasant. There is also a risk that pulses of high intensity light can temporarily or permanently damage the eyes.

The pulses provided by conventional consumer devices for hair growth reduction are in general of such intensity and optical spectrum that they can cause after images in the vision of the user, which can be unpleasant for the user. In addition, pulses from these devices are typically short. Mostly they are generated by a free discharge of a capacitor over a gas discharge lamp and have a full-width-at-half-maximum on the order of a millisecond.

### SUMMARY OF THE INVENTION

It is also known that the duration of a typical blink of an eye is significantly longer than the duration of a light pulse generated by a consumer grade hair removal device. The duration of an eye blink is dependent on the circumstances (e.g. normal eyes or dry eyes, the type of activity that the subject is doing). A typical minimum duration of a blink can be of the order of 0.05 - 0.1 second.

Thus, it can be seen from a comparison of the duration of a typical pulse of a consumer grade light-pulse-based device to the duration of an eye blink, it is possible for the light pulse to be emitted during the time that the eye is blinking (e.g. completely closed or substantially closed), or otherwise closed, reducing the discomfort for the user of the device and/or reducing the risk of damage to the eyes of the user.

Therefore according to a first specific aspect, there is provided an apparatus for controlling the generation of a light pulse by a device. The device is configured to apply the light pulse to skin of a subject to perform a treatment operation on or to the skin, and the apparatus comprises a processing unit configured to receive a first measurement signal from a first sensor configured to monitor at least one eye of a user of the device during use of the device; process the first measurement signal to determine an amount of closure of the at least one eye of the user; determine whether each of one or more criteria is satisfied, said one or more criteria comprising at least a closure criterion requiring the at least one eye of the user to be closed by at least a minimum amount of closure; and activate the device to generate a light pulse if it is determined that each of said one or more criteria is satisfied. Thus, the apparatus ensures that the eye(s) of the user are closed by at least the minimum amount of closure before a light pulse is generated, which reduces the discomfort for the user of the device by the light pulse and/or reduces the risk of damage to the eyes of the user by the light pulse.

In some embodiments the processing unit is configured to determine that the device is not to generate a light pulse if it is determined that any of the one or more criteria are not satisfied.

In some embodiments the one or more criteria further comprise a contact criterion requiring the device to be in contact with the skin. In this way, a light pulse can only be generated if the device is in contact with the skin, reducing the risk that the user, or another person, could look directly at the light pulse when it is generated.

In these embodiments the processing unit can be further configured to receive a second measurement signal from a second sensor that measures contact between the device and skin; and process the second measurement signal to determine if the device is in contact with the skin.

In some embodiments the one or more criteria further comprise a skin tone criterion requiring a skin tone of the skin to be either: below a skin tone threshold, above a skin tone threshold, or in a skin tone threshold range. This has the advantage that the light pulse can only be generated if it is to be applied to skin having a suitable skin tone.

In these embodiments the processing unit can be further configured to determine if the skin tone criterion is satisfied by processing the first measurement signal to determine a skin tone or processing a third measurement signal received from a third sensor that measures skin tone. The former option has the advantage that no additional sensor is required in order to assess the skin tone criterion.

In some embodiments the one or more criteria further comprise a charged criterion requiring a component in the device for causing a light source in the device to generate the light pulse to be charged.

In these embodiments the processing unit can be further configured to receive a charge status signal from the device; and process the charge status signal to determine if the component is charged.

In some embodiments the one or more criteria further comprise a trigger criterion requiring a pulse trigger to be provided by the user. This has the advantage that a positive action is required by the user before a light pulse can be generated.

In these embodiments the one or more criteria can further comprise a timer criterion requiring a first timer not to have expired, wherein the first timer is started after the pulse trigger is provided by the user. The timer used in these embodiments provides a typically short period of time after a pulse trigger is provided by the user for the user to satisfy the closure criterion and for a light pulse to be generated.

In these embodiments the processing unit can be further configured to start a second timer after the pulse trigger is provided by the user, wherein the second timer is set to expire at the same time as the first timer, and the second timer has a shorter duration than the first timer; and activate the device to generate a light pulse if it is determined that a light pulse has not been generated since the pulse trigger was provided by the user and the second timer has been started. These embodiments have the benefit that even if the closure criterion is not met by the time that the second timer is started, a light pulse can still be generated. This can avoid frustration for the user in trying to operate the device.

In these embodiments the processing unit can be configured to activate the device to generate a light pulse if it is determined that a light pulse has not been generated since the pulse trigger was provided by the user, the second timer has been started and the at least one of the contact criterion, the skin tone criterion and the charged criterion are satisfied.

In some embodiments the minimum amount of closure is one of: a pupil of an eye completely covered by an eyelid, at least 50% closed, at least 60% closed, at least 70% closed, at least 75% closed, at least 80% closed, at least 90% closed, and 100% closed.

In some embodiments the apparatus further comprises the first sensor.

According to a second aspect, there is provided a system for performing a treatment operation on or to skin of a subject. The system comprises a device for use by a user, wherein the device is for generating a light pulse that is to be applied to the skin of the subject to perform the treatment operation; a first sensor for monitoring at least one eye of the user; and an apparatus according to the first aspect or any embodiment thereof for controlling the generation of the light pulse by the device.

In some embodiments the apparatus is part of the device. In alternative embodiments, the apparatus is separate from the device.

According to a third aspect, there is provided a method for controlling the generation of a light pulse by a device. The device is configured to apply the light pulse to skin of a subject to perform a treatment operation on or to the skin, and the method comprises receiving a first measurement signal from a first sensor configured to monitor at least one eye of a user of the device during use of the device; processing the first measurement signal to determine an amount of closure of the at least one eye of the user; determining whether each of one or more criteria is satisfied, said one or more criteria comprising at least a closure criterion requiring the at least one eye of the user to be closed by at least a minimum amount of closure; and activating the device to generate a light pulse if it is determined that each of said one or more criteria is satisfied. Thus, the method ensures that the eye(s) of the user are closed by at least the minimum amount of closure before a light pulse is generated, which reduces the discomfort for the user of the device by the light pulse and/or reduces the risk of damage to the eyes of the user by the light pulse.

In some embodiments the method further comprises determining that the device is not to generate a light pulse if it is determined that any of the one or more criteria are not satisfied.

In some embodiments the one or more criteria further comprise a contact criterion requiring the device to be in contact with the skin. In this way, a light pulse can only be generated if the device is in contact with the skin, reducing the risk that the user, or another person, could look directly at the light pulse when it is generated.

In these embodiments the method can further comprise receiving a second measurement signal from a second sensor that measures contact between the device and skin; and processing the second measurement signal to determine if the device is in contact with the skin.

In some embodiments the one or more criteria further comprise a skin tone criterion requiring a skin tone of the skin to be either: below a skin tone threshold, above a skin tone threshold, or in a skin tone threshold range. This has the advantage that the light pulse can only be generated if it is to be applied to skin having a suitable skin tone.

In these embodiments the step of determining whether each of one or more criteria is satisfied further comprises determining if the skin tone criterion is satisfied by processing the first measurement signal to determine a skin tone or processing a third measurement signal received from a third sensor that measures skin tone. The former option has the advantage that no additional sensor is required in order to assess the skin tone criterion.

In some embodiments the one or more criteria further comprise a charged criterion requiring a component in the device for causing a light source in the device to generate the light pulse to be charged.

In these embodiments the method can further comprise receiving a charge status signal from the device; and processing the charge status signal to determine if the component is charged.

In some embodiments the one or more criteria further comprise a trigger criterion requiring a pulse trigger to be provided by the user. This has the advantage that a positive action is required by the user before a light pulse can be generated.

In these embodiments the one or more criteria can further comprise a timer criterion requiring a first timer not to have expired, wherein the first timer is started after the pulse trigger is provided by the user. The timer used in these embodiments provides a typically short period of time after a pulse trigger is provided by the user for the user to satisfy the closure criterion and for a light pulse to be generated.

In these embodiments the method can further comprise starting a second timer after the pulse trigger is provided by the user, wherein the second timer is set to expire at the same time as the first timer, and the second timer has a shorter duration than the first timer; and activating the device to generate a light pulse if it is determined that a light pulse has not been generated since the pulse trigger was provided by the user and the second timer has been started. These embodiments have the benefit that even if the closure criterion is not met by the time that the second timer is started, a light pulse can still be generated. This can avoid frustration for the user in trying to operate the device.

In these embodiments the method can further comprise activating the device to generate a light pulse if it is determined that a light pulse has not been generated since the pulse trigger was provided by the user, the second timer has been started and the at least one of the contact criterion, the skin tone criterion and the charged criterion are satisfied.

In some embodiments the minimum amount of closure is one of: a pupil of an eye completely covered by an eyelid, at least 50% closed, at least 60% closed, at least 70% closed, at least 75% closed, at least 80% closed, at least 90% closed, and 100% closed.

According to a fourth aspect, there is provided a computer program product comprising a computer readable medium having computer readable code embodied therein, the computer readable code being configured such that, on execution by a suitable computer or processor, the computer or processor is caused to perform the method according to the third aspect or any embodiment thereof.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is an illustration of an exemplary device for generating light pulses;
Fig. 2 is a block diagram of an exemplary system comprising a device and an apparatus according to an embodiment;
Fig. 3 is a flow chart illustrating a method according to an exemplary embodiment;
Fig. 4 is a set of illustrations showing an eye in various states of closure; and
Fig. 5 shows several exemplary trigger signals.

### DETAILED DESCRIPTION OF EMBODIMENTS

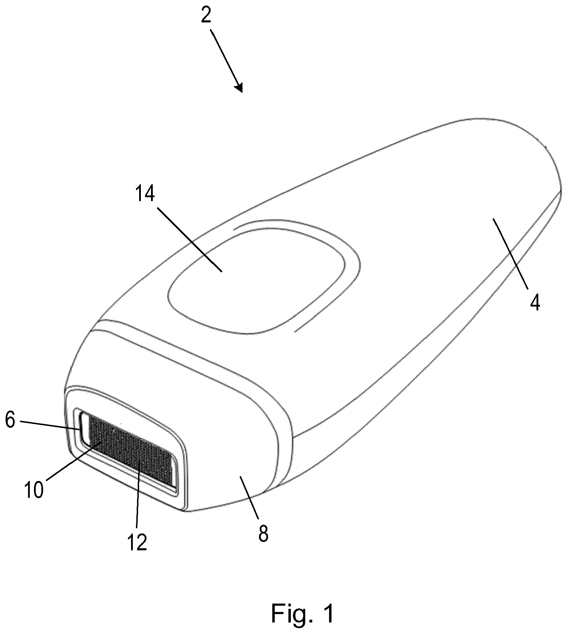

Fig. 1 is an illustration of an exemplary device 2. The techniques described herein can be used to control the generation of light pulses by the device 2 that are applied to skin of a subject to perform an operation on the skin or body of the subject. The device 2 is for use on a body of a subject (e.g. a person or an animal), and maybe a hand-held device that is to be held in one or both hands of a user during use. In some embodiments (depending on the operation to be performed), the device 2 can perform the operation on the skin or body of the subject through the clothing of the subject (i.e. the device 2 can be in contact with the skin or body via the subject's clothing), and, where possible, references herein to contact with the body or skin should be understood as including, or potentially including, contact with the body or skin via one or more layers of clothing).

As described herein, the device 2 is operated or used by a 'user', and the device 2 is used on a body of a 'subject'. In some cases the user and the subject is the same person, i.e. the device 2 is held in a hand and used by the subject on themselves (e.g. used on the skin on their leg). In other cases the user and the subject are different people, e.g. the device 2 is held in a hand and used by a user on someone else.

The device 2 comprises a housing 4 that includes a head portion 6 at one end 8 of the body 4 (referred to herein as the 'head end' of the device 2), which is the end of the device 2 that is to be placed into contact with the subject and the end from which the light pulse is emitted.

In some embodiments the device 2 is for performing a treatment operation or a personal care operation on the body using light pulses, the head portion 6 can be referred to as a 'treatment head portion 6', and the treatment head portion 6 will include suitable components for enabling the light pulse to be generated.

Some exemplary treatment operations or a personal care operations include, but are not limited to, the removal of unwanted hairs by laser and light therapies (known as photoepilation); a cosmetic or medical dermatological (skin) treatment, including hair growth reduction, treating acne, treatment of spider veins (telangiectasia) or varicose veins, a phototherapy treatment, skin rejuvenation, skin tightening, or port-wine stain treatment.

The head portion 6 of the device 2 comprises an aperture 10 that is arranged in or on the housing 4 so that the aperture 10 can be placed adjacent to or on (i.e. in contact with) the skin (or clothing) of the subject. The device 2 includes one or more light sources 12 that are for generating light pulses that are to be applied to the skin of the subject via the aperture 10 and effect a treatment operation. The one or more light sources 12 are arranged in the housing 4 so that the light pulses are provided from the one or more light sources 12 through the aperture 10. The aperture 10 may be in the form of an opening at one end of the housing 4, or it may be in the form of a window (including a waveguide) that is transparent or semi-transparent to the light pulses (i.e. the light pulses can pass through the window).

In the exemplary embodiment shown in Fig. 1, the aperture 10 has a generally rectangular shape, which results in a generally rectangular-shaped skin treatment region on the skin. It will be appreciated that the aperture 10 can have any other desired shape. For example the aperture 10 can be square, elliptical, circular, or any other polygonal shape. Moreover, it will be appreciated that the aperture 10 may be flat (i.e. two-dimensional), or have a three-dimensional configuration (e.g. curved).

The one or more light sources 12 can generate light pulses of any suitable or desired wavelength (or range of wavelengths) and/or intensities. For example, the light source(s) 12 can generate visible light, infra-red (IR) light and/or ultraviolet (UV) light. The light source(s) 12 can be any of one or more light emitting diodes (LEDs), one or more flash lamps, e.g. Xenon flash lamps or Krypton flash lamps, one or more lasers, etc.

The light pulses provided by the light source(s) 12 typically have a short duration, e.g. much less than 1 second, and typically shorter than the typical duration of a blink of an eye by a person (e.g. a typical blink duration can be 0.1 second). In the case of consumer devices for use in the home (for example), the pulse duration may typically be of the order of 1 millisecond (ms) or 2 ms. These pulse durations are based on a full width at half maximum (FWHM) of a free discharge. According to alternative definitions, the pulse duration could be (significantly) longer, e.g. 10 ms or more. In the case of devices for use by treatment professionals (e.g. healthcare professionals), the pulse duration (in terms of FWHM) may be significantly longer (10s of milliseconds, up to a few 100s of milliseconds).

Finally in Fig. 1, the illustrated device 2 includes a user control 14 that can be operated by the user to trigger or start the generation of a light pulse by the device 2. The user control 14 may be in the form of a switch, a button, a touch pad, a trigger, etc.

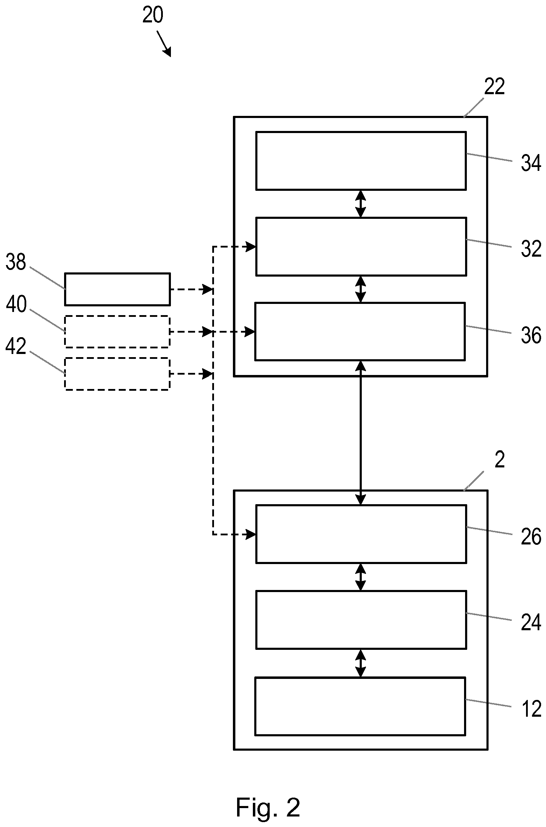

Fig. 2 is a block diagram of an exemplary system 20 that comprises a device 2 as described above and an apparatus 22 for implementing exemplary embodiments of the techniques described herein. In this illustrated embodiment, the apparatus 22 is shown as being physically separate from the device 2, but it will be appreciated that in some embodiments of the system 20 the apparatus 22, or the functionality provided by the apparatus 22, can be part of, or integral with, the device 2.

In Fig. 2, the device 2 is shown as comprising one or more light sources 12 for generating light pulses and light source control circuitry 24. The light source control circuitry 24 is for controlling and enabling the generation of light pulses by the light source(s) 12, and the light source control circuitry 24 can comprise one or more components (e.g. capacitors) that are to be charged with electrical energy, and that are discharged across the light source(s) 12 to cause the light source(s) 12 to generate a light pulse. In the illustrated embodiment, as the device 2 is separate from the apparatus 22, the device 2 also comprises interface circuitry 26. The interface circuitry 26 enables information, including measurements by sensors in the device 2, to be communicated to the apparatus 22, and/or enables information, including control signals for the device 2, to be communicated from the apparatus 22 to the device 2. In embodiments where the apparatus 22, or the functionality provided by the apparatus 22 is part of or integral with the device 2, the interface circuitry 26 may not be required.

The apparatus 22 comprises a processing unit 32 that is for controlling the generation of a light pulse by the device 2 according to the techniques described herein. The processing unit 32 can be implemented in numerous ways, with software and/or hardware, to perform the various functions described herein. The processing unit 32 may comprise one or more microprocessors or digital signal processor (DSPs) that maybe programmed using software or computer program code to perform the required functions and/or to control components of the processing unit 32 to effect the required functions. The processing unit 32 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, microcontrollers, DSPs and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

In some embodiments the light source control circuitry 24 can be implemented according to the options and examples of the processing unit 32 as described above.

The processing unit 32 is connected to a memory unit 34 (in alternative embodiments the memory unit 34 can be part of the processing unit 32), and the memory unit 34 can store data, information and/or signals for use by the processing unit 32 in controlling the generation of a light pulse by the device 2. In some implementations the memory unit 34 stores computer-readable code that can be executed by the processing unit 32 so that the processing unit 32 performs one or more functions, including the operations described herein. The memory unit 34 can comprise any type of non-transitory machine-readable medium, such as cache or system memory including volatile and non-volatile computer memory such as random access memory (RAM) static RAM (SRAM), dynamic RAM (DRAM), read-only memory (ROM), programmable ROM (PROM), erasable PROM (EPROM) and electrically erasable PROM (EEPROM), implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

As noted above, in the illustrated embodiment the device 2 is separate from the apparatus 22 and includes interface circuitry 26. The apparatus 22 therefore includes interface circuitry 36 that enables the apparatus 22 to communicate with the device (e.g. to receive measurements by sensors in the device 2 and/or to send control signals to the device 2). In embodiments where the apparatus 22, or the functionality provided by the apparatus 22, is part of or integral with the device 2, the interface circuitry 36 in the apparatus 22 may be omitted.

The interface circuitry 26 in the device 2 and the interface circuitry 36 in the apparatus 22 are each for enabling a data connection to and/or data/information exchange with another device/apparatus/unit. The connection between the device 2 and the apparatus 22 maybe direct or indirect, e.g. via the Internet, including via an intermediate electronic device such as a smartphone, tablet or laptop, and thus the interface circuitry 26, 36 can enable a connection between the device 2/apparatus 22 and a network, such as the Internet, or directly between the apparatus 22 and device 2, via any desirable wired or wireless communication protocol. For example, the interface circuitry 26, 36 can operate using WiFi, Bluetooth, Zigbee, or any cellular communication protocol (including but not limited to Global System for Mobile Communications (GSM), Universal Mobile Telecommunications System (UMTS), Long Term Evolution (LTE), LTE-Advanced, etc.). In the case of a wireless connection, the interface circuitry 26, 36 (and thus the respective one of the device 2 and apparatus 22) may include one or more suitable antennas for transmitting/receiving over a transmission medium (e.g. the air). Alternatively, in the case of a wireless connection, the interface circuitry 26, 36 may include means (e.g. a connector or plug) to enable the interface circuitry 26, 36 to be connected to one or more suitable antennas external to the respective one of the device 2 and apparatus 10 for transmitting/receiving over a transmission medium (e.g. the air). The interface circuitry 36 is connected to the processing unit 32 to enable information or data received by the interface circuitry 36 to be provided to the processing unit 32, and/or information or data from the processing unit 32 to be transmitted by the interface circuitry 36.

As shown in Fig. 2, the system 20 also comprises a first sensor 38. The first sensor 38 is provided to monitor one or both eyes of the user and to output a first measurement signal. The first measurement signal is to be processed by the processing unit 32 to determine whether the eye or eyes of the user are closed or sufficiently closed. The first measurement signal can be a time series of measurements or measurement samples that directly or indirectly represent the closure state or amount of closure of an eye (eyelid) or both eyes (both eyelids). For example, the first sensor 38 can be a sensor that is in contact with, or positioned close to, one or both eyelids, such as an accelerometer, or a sensor that provides measurements of the electrical activity of the eye(s)/eye lid(s), such as an electrooculogram (EOG) sensor. Alternatively, the first measurement signal can be a time series of images (or a video sequence) obtained by an imaging unit that can be processed to identify the eye or eyes of the user in the image(s), and processed to determine the amount of closure of the eye(s). An imaging unit may include any suitable component for capturing an image, for example a charge-coupled device (CCD) and one or more lenses and/or mirrors. In some embodiments, the first sensor 38 is a camera, such as a digital camera.

As the measurements from the first sensor 38 are to be used to detect when the user is blinking, and a blink can have a typical duration of the order of 0.05 to 0.1 second, the sampling frequency of the first sensor 38 (i.e. the frequency with which the first sensor 38 makes a measurement) should be at least 25 Hertz (Hz) to enable blinks to be detected. In some embodiments the sampling frequency of the first sensor 38 is 50 Hz or higher. For example, in the case of an imaging unit operating at 50 Hz, a new image is available every 20 milliseconds (movement sensor 6).

Where the first sensor 38 requires contact with the user to monitor the user's eye(s), the first sensor 38 may be separate from the device 2 and separate from the apparatus 22. For example an accelerometer or EOG sensor will need to be placed in contact with the user's eyelid(s) or otherwise near the eye(s). In the case of an imaging unit, the imaging unit should be positioned or able to be positioned so that the user's face or eye(s) are in the field of view of the imaging unit, so that images of the user's face or eye(s) can be obtained. The imaging unit could therefore be part of the device 2, part of the apparatus 22 (e.g. it could be a camera or other imaging unit present in a smartphone, tablet or laptop) or separate from both the device 2 and the apparatus 22.

The first measurement signal from the first sensor 38 is to be provided to the processing unit 32, and therefore the first sensor 38 can be directly or indirectly connected to the processing unit 32, e.g. via interface circuitry 26 and/or interface circuitry 36, depending on the arrangement of the first sensor 38 relative to the device 2 and apparatus 22. Where the first sensor 38 is part of the apparatus 22, or part of the device 2 where the functionality of the apparatus 22 is provided in the device 2, the first sensor 38 can be directly connected to the processing unit 32.

It should be noted that Fig. 2 only shows the elements of a device 2 and apparatus 22 that are useful for implementing the techniques described herein, and a typical device 2 and apparatus 22 will include further elements. For example the device 2 and/or the apparatus 22 will also include a power supply, such as a battery, or elements enabling the device 2 and/or apparatus 22 to be connected to a mains power supply.

In the embodiment shown in Fig. 2 where the apparatus 22 is separate from the device 2, the apparatus 22 can be any type of electronic device or computing device that can communicate with the device 2, and receive measurements directly or indirectly from, the first sensor 38. For example the apparatus 22 can be, or be part of, a computer, a laptop, a tablet, a smartphone, a smartwatch, a base unit for the device 2, etc. In other implementations, the apparatus 22 can be an apparatus that is remote from the device 2. For example, the apparatus 22 can be a server, such as a server in a data centre (also referred to as being 'in the cloud'). In these examples, the device 2 may include the necessary functionality (e.g. the interface circuitry 26) to enable the device 2 to communicate the first measurement signal to the apparatus 22, e.g. via the Internet. Alternatively, the device 2 may communicate the first measurement signal to another electronic device such as a smartphone, tablet or laptop (for example via Bluetooth, WLAN, or any other suitable communication protocol), and that electronic device can communicate the first measurement signal to the apparatus/server 22.

In some embodiments, the system 20 can also include a second sensor 40 that is for measuring or detecting whether there is contact between the device 2 and skin. The second sensor 40 is also referred to herein as contact sensor, and the contact sensor 40 outputs a second measurement signal (or 'contact signal') comprising a time series of measurements relating to whether there is contact with the skin or body. The contact sensor 40 can be used to measure or detect whether the device 2, and for example the head portion 6, is in contact with the skin or body of the subject. The contact sensor 40 can be provided on or in the device 2, for example, positioned at or on the head portion 6 so that the contact sensor 40 is in contact with the skin or body when the device 2 is in contact with the body, and the contact sensor 40 outputs a contact signal indicating whether there is contact with the skin or body. In these embodiments, the contact sensor 40 can be any suitable type of sensor, for example a pressure sensor, a conductivity sensor, a capacitive contact sensor, a proximity sensor, an optical-based contact sensor (e.g. that can detect contact based on a measured light level), or an imaging unit, such as a camera (where the image(s) from the imaging unit can be processed to identify the subject and the device 2 and to determine if the device 2 is in contact with the subject). The contact signal may be a voltage signal that varies between high and low voltages (representing contact and no contact between the contact sensor 40 and the skin or body). In alternative embodiments, contact between the device 2 and the skin or body can be determined from images or a video sequence that shows the device 2 and the skin or body, with the image(s) being processed to identify the device, identify the skin or body and determine the relative positions thereof. In this case, where the first sensor 38 is an imaging unit, the imaging unit can be used for both monitoring the eye(s) of the user and determining whether there is contact between the device 2 and the skin or body.

The output from the contact sensor 40 is to be provided to the processing unit 32, and therefore the contact sensor 40 can be directly or indirectly connected to the processing unit 32, e.g. via interface circuitry 26 and/or interface circuitry 36, depending on the arrangement of the contact sensor 40 relative to the device 2 and apparatus 22. Where the contact sensor 40 is part of the apparatus 22, or part of the device 2 where the functionality of the apparatus 22 is provided in the device 2, the contact sensor 40 can be directly connected to the processing unit 32.

In some embodiments, the system 20 can also include a third sensor 42 that is for measuring or detecting the skin tone of the skin that the device 2, for example the head portion 6, is in contact with. The skin tone can be measured in terms of an amount of melanin in the skin or a melanin index, for example. The third sensor 42 is also referred to herein as a 'skin tone sensor'. The skin tone sensor 42 can be provided on or in the device 2, for example, positioned at or on the head portion 6. The skin tone sensor 42 can output a third measurement signal (or 'skin tone signal') comprising a time series of measurements relating to the skin tone of the skin. The skin tone sensor 42 can measure the skin tone directly, or it can measure the skin tone indirectly, for example by measuring a feature or parameter that is related to skin tone, and analysing the feature or parameter value to infer the skin tone of the skin. The skin tone sensor 42 can be any suitable type of sensor, for example an imaging unit, such as a camera or an arrangement that measures the reflectance of the skin at one or more wavelengths. In the case of an imaging unit, the skin tone (or skin tone signal) can be determined from images or a video sequence that shows the skin to be treated, with the image(s) being processed to determine the skin tone. In this case, where the first sensor 38 is an imaging unit, the imaging unit can be used for both monitoring the eye(s) of the user and determining the skin tone, or separate imaging units can be provided for monitoring the eye(s) of the user and detecting the skin tone of the subject.

The output from the skin tone sensor 42 is to be provided to the processing unit 32, and therefore the skin tone sensor 42 can be directly or indirectly connected to the processing unit 32, e.g. via interface circuitry 26 and/or interface circuitry 36, depending on the arrangement of the skin tone sensor 42 relative to the device 2 and apparatus 22. Where the skin tone sensor 42 is part of the apparatus 22, or part of the device 2 where the functionality of the apparatus 22 is provided in the device 2, the skin tone sensor 42 can be directly connected to the processing unit 32.

In various embodiments, the system 20 can comprise one, both, or neither of the second sensor 40 and the third sensor 42.

As noted above, the pulses provided by conventional consumer devices for hair growth reduction are in general of such intensity and optical spectrum that they can cause after images in the vision of the user, which is unpleasant and disturbs the user, and/or the light pulses may damage the eyes/vision of the user. However, it is also known that the duration of a typical blink of an eye (of the order of 0.05 to 0.1 second) is significantly longer than the duration of a light pulse generated by a consumer grade hair removal device (of the order of 1 millisecond), and therefore the techniques described herein provide that the eye or eyes of the user are monitored, and a light pulse generated when the eye or eyes is closed by at least a minimum amount (e.g. due to blinking). In this way, the amount of light from the light pulse entering the eye(s) of the user is reduced as the eye(s) is/are closed when the light pulse is generated, reducing the occurrence of after images in the user's vision, improving the user's experience of performing the treatment operation using the device 2, and/or improving the safety of the device 2.

In certain embodiments, in addition to the requirement that the at least one eye is closed by at least a minimum amount, one or more additional criteria also have to be satisfied in order for a light pulse to be triggered. These additional criteria are described in more detail below, and can relate to whether the device 2 is in contact with the skin, the skin tone of the skin that the device 2 is in contact with, and the timing of a user providing a light pulse trigger.

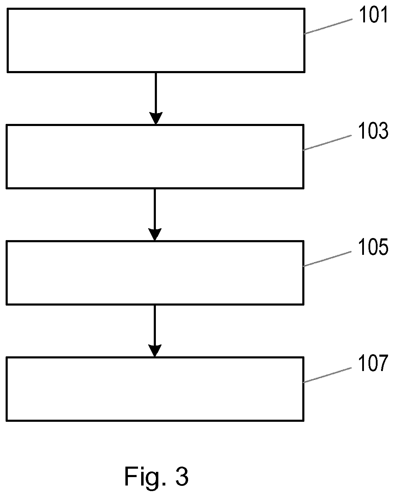

The flow chart in Fig. 3 illustrates an exemplary method for controlling the generation of a light pulse by a device 2 according to the techniques described herein. The light pulse is to be applied to skin of a subject in order to perform a treatment operation on or to the skin, such as hair removal, treating acne, a phototherapy treatment, skin rejuvenation, skin tightening, or port-wine stain treatment, etc.

One or more of the steps of the method (although not necessarily all steps) can be performed by the processing unit 32 in the apparatus 22, in conjunction with any of the memory unit 34, interface circuitry 36 and first sensor 38 as appropriate. The processing unit 32 may perform the one or more steps in response to executing computer program code, that can be stored on a computer readable medium, such as, for example, the memory unit 34.

In a first step, step 101, the processing unit 32 receives a first measurement signal from the first sensor 38. The first sensor 38 is configured to monitor one or both eyes of the user of the device 2 during use of the device 2, and the first measurement signal represents measurements of the one or both eyes, and in particular enables an amount of closure of the eye or eyes to be determined. As noted above, in some embodiments the first sensor 38 can be an accelerometer, an EOG sensor or an imaging unit, and thus the first measurement signal can be an acceleration signal, an EOG signal or a series of images (video sequence). The first measurement signal can be received by the processing unit 32 directly from the first sensor 38 or indirectly from the first sensor 38 (e.g. via interface circuitry 36 and potentially interface circuitry 26 as well).

The techniques described herein provide that the eye or eyes of the user are monitored and a light pulse generated when the eye or eyes is closed by at least a minimum amount. As the duration of the eye closure due to blinking may be of the order of 0.05 to 0.1 seconds, the processing unit 32 receives the first measurement signal in real-time (i.e. as the measurements are made) or in near-real-time (i.e. with only a delay of the order of a few milliseconds from when the measurements were made), and performs the subsequent steps of the method to determine if a light pulse is to be generated during that blink over a timescale that is less than the duration of a blink.

In step 103, the processing unit 32 processes the first measurement signal to determine an amount of closure of the at least one eye of the user. As noted above, the first measurement signal can be a time series of measurements or measurement samples that directly or indirectly represent the closure state or amount of closure of an eye (eyelid) or both eyes (both eyelids). The processing used in step 103 to determine the amount that the eye(s) are closed will depend on the type of first sensor 38 present in the system 20, and those skilled in the art will be aware of various techniques for determining an amount that an eye is closed from a first measurement signal, such as an acceleration signal for an accelerometer located near to an eye or eyelid, an EOG signal, or a series of images of the user's face or eye(s). For example, suitable techniques can include those used in digital cameras for detecting a blink by a subject in an image.

The processing unit 32 can process the first measurement signal to determine the amount of closure of the at least one eye, with the amount of closure being represented as a percentage or proportion that the eye is closed.

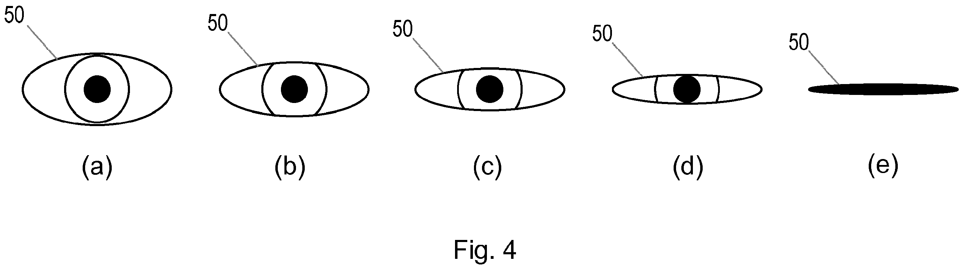

Fig. 4 illustrates an eye 50 in various states of 'closure' (or openness). In Fig. 4(a), the eye 50 is fully open, so 0% closed. In Fig. 4(b), the eye 50 is 75% open, so 25% closed. In Fig. 4(c), the eye 50 is half (50%) open, so half (50%) closed. In Fig. 4(d), the eye 50 is 25% open, so 75% closed. In Fig. 4(e), the eye 50 is fully (100%) closed, so 0% open.

In some embodiments step 103 comprises determining the amount of closure of just one eye of the user, and using this amount of closure in subsequent steps of the method. In alternative embodiments, step 103 comprises separately determining the amount of closure of each eye of the user, and using these separate amounts of closure in subsequent steps of the method. In other embodiments, step 103 comprises separately determining the amount of closure of each eye of the user, combining these amounts (e.g. by averaging the amounts, taking the largest amount or taking the smallest amount) into a single amount of closure that is used in subsequent steps of the method.

Next, in step 105, the processing unit 32 determines whether each of one or more criteria is satisfied. The one or more criteria relate to conditions that are each to be satisfied in order for a light pulse to be generated by the device 2. The one or more criteria comprises at least a closure criterion that relates to the amount of closure of the eye or eyes of the user. In particular, the closure criterion is satisfied if the at least one eye of the user is closed by at least a minimum amount of closure. In some embodiments step 105 comprises comparing the amount of closure determined in step 103 to a closure threshold. The closure criterion can be satisfied where the amount of closure is equal to or above the closure threshold. For example the closure threshold can be 100% or another measure representing the eye(s) being fully closed, such as the pupil of the eye being completely covered by the eyelid, in which case the closure criterion will not be satisfied if the eye(s) are partly open/at least part of the pupil is not covered by the eyelid. In this example only the eye state shown in Fig. 4(e) would meet the closure criterion. As another example, the closure threshold can be 60% in which case the closure criterion will not be satisfied if the eye(s) are open by more than 40%. In this example only the eye states shown in Figs. 4 (d) and 4(e) would meet the closure criterion. Those skilled in the art will appreciate that the closure threshold can be set to any desired or suitable value, with higher values of the closure threshold being preferred. For example, the closure threshold can be any of 50%, 60%, 70%, 75%, 80%, 90%, and 100%.

Further criteria that can be evaluated in step 105 are described in more detail below.

If it is determined in step 105 that all of the one or more criteria are satisfied, then the device 2 can be activated to generate a light pulse (step 107). This step can comprise the processing unit 32 providing a suitable control signal to the light source control circuitry 24 in order to cause the light source control circuitry 24 to control the light source(s) 12 to generate a light pulse. Depending on the relative arrangement of the processing unit 32 and the light source control circuitry 24 in the device 2 (e.g. the processing unit 32 can be in a separate apparatus 22 or part of the device 2), the processing unit 32 may provide the control signal to the light source control circuitry 24 via the interface circuitry 36 and interface circuitry 26. Since the light pulse should be generated while the eye(s) of the user are closed or sufficiently closed, the device 2 is to be activated to generate the light pulse while all the one or more criteria (and particularly the closure criterion) are satisfied.

On receipt of the control signal, the light source control circuitry 24 can cause the light source(s) 12 to generate a light pulse. For example the light source control circuitry 24 can discharge a capacitor into the light source(s) 12 so that the light source(s) 12 generate a light pulse.

If it is determined in step 105 that at least one of the criteria is not satisfied (e.g. the closure criterion is not satisfied as the eye(s) are closed less than the closure threshold), then the processing unit 32 determines that the device 2 is not to generate a light pulse at this stage. In this case a control signal that can trigger the generation of a light pulse is not sent to the light source control circuitry 24.

In some embodiments the criteria evaluated in step 105 further comprises a contact criterion in addition to the closure criterion. The contact criterion requires the device 2 to be in contact with the skin, and the contact criterion is satisfied if the device 2 is in contact with the skin. In these embodiments, the device 2 is activated to generate a light pulse in step 107 if both the closure criterion and the contact criterion are satisfied. However, if one or both of the closure criterion and the contact criterion are not satisfied, then the device 2 is not activated and no light pulse is generated.

In these embodiments, to enable the processing unit 32 to evaluate the contact criterion, the system 20 includes the second sensor 40 (contact sensor) that measures contact between the device 20 and skin. The processing unit 32 receives a second measurement signal (contact signal) from the contact sensor 40 and processes the contact signal to determine if the device 2 is in contact with the skin. If the contact signal indicates that the device 2 is in contact with the skin, then the contact criterion is satisfied. If the contact signal indicates that the device 2 is not in contact with the skin, then the contact criterion is not satisfied.

In some embodiments the criteria evaluated in step 105 further comprises a skin tone criterion in addition to the closure criterion. In some embodiments, the skin tone criterion requires the skin tone of the skin that the device 2 is in contact with to be below a skin tone threshold. The skin tone threshold relates to skin tones that are suitable for treatment by the light pulse. The skin tone criterion is satisfied if the skin tone is below the skin tone threshold. In these embodiments the skin tone threshold could be provided in terms of a value of the Melanin Index, e.g. a Melanin Index of 350 or 450, or another value in the range of 1 to 999. In alternative embodiments, the skin tone criterion requires the skin tone of the skin that the device 2 is in contact with to be above a skin tone threshold. The skin tone threshold relates to skin tones that are suitable for treatment by the light pulse. The skin tone criterion is satisfied if the skin tone is above the skin tone threshold. In other alternative embodiments, the skin tone criterion requires the skin tone of the skin that the device 2 is in contact with to be in a skin tone range (i.e. the skin tone of the skin should be above a lower skin tone threshold and below an upper skin tone threshold). The skin tone range relates to a range of skin tones that are suitable for treatment by the light pulse. The skin tone criterion is satisfied if the skin tone is in the skin tone range. In any of these embodiments, the device 2 is activated to generate a light pulse in step 107 if both the closure criterion and the skin tone criterion are satisfied. However, if one or both of the closure criterion and the skin tone criterion are not satisfied, then the device 2 is not activated and no light pulse is generated.

In these embodiments, to enable the processing unit 32 to evaluate the skin tone criterion, the system 20 can either include the third sensor 42 (skin tone sensor) that measures the skin tone of the skin, or the processing unit 32 can determine the skin tone from the first measurement signal from the first sensor 38, particularly in the case where the first sensor 38 is an imaging unit. The processing unit 32 either processes the first measurement signal to determine the skin tone, or receives the third measurement signal (skin tone signal) from the skin tone sensor 42 and processes the skin tone signal to determine the skin tone. If the determined skin tone indicates that the skin tone is below the skin tone threshold, then the skin tone criterion is satisfied. If the determined skin tone indicates that the device 2 is not below the skin tone threshold, then the skin tone criterion is not satisfied.

In some embodiments the criteria evaluated in step 105 can include both the contact criterion and the skin tone criterion. In this case, the device 2 is activated to generate a light pulse in step 107 if all the closure criterion, the contact criterion and the skin tone criterion are satisfied, i.e. the device 2 is activated if the user's eye or eyes are closed or sufficiently closed, the device 2 is in contact with the skin, and the skin is an acceptable tone. However, if any one or more of the closure criterion, the contact criterion and the skin tone criterion are not satisfied, then the device 2 is not activated and no light pulse is generated.

In some embodiments the criteria evaluated in step 105 further comprises a charged criterion in addition to the closure criterion. The charged criterion requires a component in the device 2 that causes the light source(s) 12 to generate the light pulse to be charged with electrical energy. In other words, the charged criterion relates to whether the device 2 is ready to generate the light pulse. The component in the device 2 can be the light source control circuitry 24, or one or more capacitors in the light source control circuitry 24. The charged criterion is satisfied if the device 2 is ready to generate the light pulse, and not satisfied if the device 2 is not ready to generate the light pulse. In these embodiments, the device 2 is activated to generate a light pulse in step 107 if both the closure criterion and the charged criterion are satisfied. However, if one or both of the closure criterion and the charged criterion are not satisfied, then the device 2 is not activated and no light pulse is generated. Indeed, it will be appreciated that if the charged criterion is not satisfied, the device 2 may not be capable of generating the light pulse in any case.

In these embodiments, to enable the processing unit 32 to evaluate the charged criterion, the processing unit 32 can be configured to receive a charge status signal from the device 2, e.g. from the light source control circuitry 24 in the device 2. The charge status signal indicates whether or not the component or light source control circuitry 24 is charged and ready to generate a light pulse. In embodiments where the apparatus 22 is separate from the device 2, the charge status signal can be provided to the apparatus 22 and processing unit 32 via the interface circuitry 36 and interface circuitry 26.

In some embodiments the criteria evaluated in step 105 can also include one or both of the contact criterion and the skin tone criterion. In this case, the device 2 is activated to generate a light pulse in step 107 if all of the criteria are satisfied. However, if any one or more of these criteria is not satisfied, then the device 2 is not activated and no light pulse is generated.

In some embodiments the criteria evaluated in step 105 further comprises a trigger criterion in addition to the closure criterion. The trigger criterion requires a pulse trigger to be provided by the user of the device 2. The pulse trigger can be provided by the user by actuating the user control 14, e.g. by the user pressing a switch 14, pulling a trigger 14, pressing a button 14, etc. In these embodiments, the device 2 is activated to generate a light pulse in step 107 if both the closure criterion and the trigger criterion are satisfied. However, if one or both of the closure criterion and the trigger criterion are not satisfied, then the device 2 is not activated and no light pulse is generated. In other words, a light pulse is generated if the user is blinking (or otherwise has their eyes closed) when a pulse trigger has been provided by the user. This means that, even if the trigger criterion is satisfied, if the user does not blink or otherwise close their eye(s) while the trigger criterion is satisfied, no light pulse is generated.

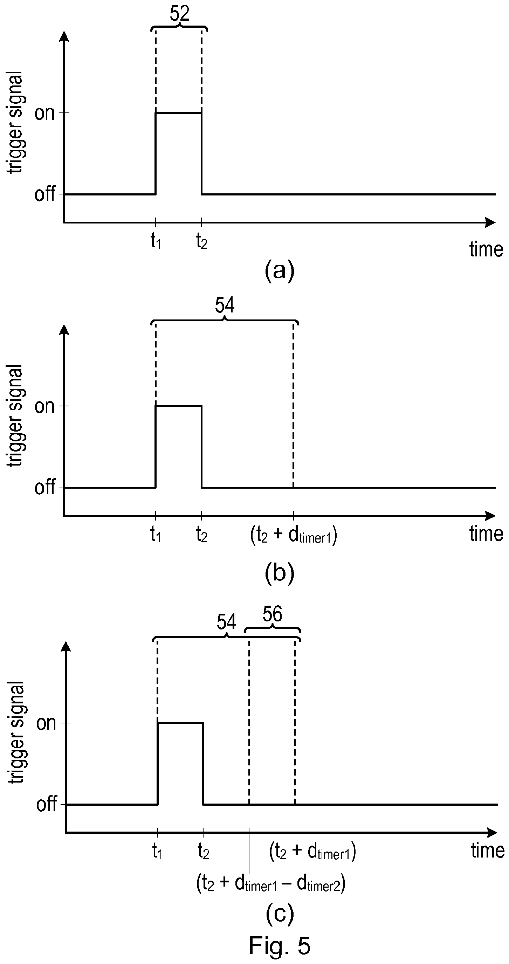

In some embodiments of the trigger criterion, the trigger criterion is satisfied if, at the time that the trigger criterion is evaluated, a pulse trigger is being provided by the user. In other words, the trigger criterion is satisfied while the user is actuating the user control 14. This is illustrated in Fig. 5(a) which shows an exemplary trigger signal provided by the user control 14. It can be seen that at time t₁, the user actuates the user control 14, and the user control 14 remains actuated until time t₂. Time t₂ is the time that the user stops actuating the user control 14 (e.g. stops pressing switch 14, stops pulling trigger 14, stops pressing button 14, etc.). Thus, in these embodiments, the trigger criterion is satisfied between times t₁ and t₂ (indicated as period 52), and so a light pulse can be generated during period 52 if at some point during this period 52 the closure criterion is also satisfied. Outside of these times (i.e. outside of period 52), the trigger condition is not satisfied and no light pulse is generated.

It will be appreciated that the length of period 52 may be quite short where the user briefly actuates the user control 14 (e.g. 1 second or less where the user presses and releases button 14), or quite long where the user actuates the user control 14 for some time (e.g. holding down a trigger 14). The latter may occur where the device 2 is used in a so-called 'slide and flash' mode where the user moves the device 2 across the skin while keeping the user control 14 actuated (e.g. moving the device 2 while keeping the button 14 pressed). In this case light pulses may be provided multiple times during a single actuation of the user control 14.

In alternative embodiments of the trigger criterion, the trigger criterion is satisfied if, at the time that the trigger criterion is evaluated, a pulse trigger is being provided by the user, or has been provided by the user no more than a predetermined time ago. In other words, the trigger criterion is satisfied while the user is actuating the user control 14 or within a time period following the actuation. This is illustrated in Fig. 5(b) which shows an exemplary trigger signal provided by the user control 14. It can be seen that at time t₁, the user actuates the user control 14, and the user control 14 remains actuated until time t₂. Time t₂ is the time that the user stops actuating the user control 14 (e.g. stops pressing switch 14, stops pulling trigger 14, stops pressing button 14, etc.). At time t₂ a timer (also referred to herein as a 'first timer') is started that has a duration denoted dₜᵢₘₑᵣ₁, and thus the first timer expires at a time t₂ + dₜᵢₘₑᵣ₁. In these embodiments, the trigger criterion is satisfied between times t₁ and t₂, and remains satisfied while the first timer is running (i.e. not expired). Thus, the trigger criterion is satisfied between times t₁ and t₂ + dₜᵢₘₑᵣ₁, i.e. in period 54, and so a light pulse can be generated during period 54 if at some point during this period 54 the closure criterion is also satisfied. Outside of these times (i.e. outside of period 54), the trigger condition is not satisfied and no light pulse is generated. The duration of the first timer can be based on an acceptable amount of time after a pulse trigger is provided by a user that the device 2 can generate the light pulse. For example, it may not be acceptable for a light pulse to be generated more than 0.5 s after the user stops actuating a user control 14. In particular embodiments, the duration of the first timer can be 100 ms or of the order of 100 ms. In some cases the maximum permitted delay for a light pulse to be generated after a user stops actuating a user control 14 may be set by local regulations or laws relating to the safe use and operation of the device 2.

In further embodiments of the trigger criterion, an 'override' can be provided that allows the closure criterion to be evaluated along with the trigger criterion to determine if a light pulse is to be generated, but the 'override' provides that a light pulse is generated in any case if the closure criterion is not satisfied within a certain amount of time. This is illustrated in Fig. 5(c) which is based on the same trigger criterion as in Fig. 5(b). Thus, Fig. 5(c) shows an exemplary trigger signal provided by the user control 14 where at time t₁ the user actuates the user control 14 and the user control 14 remains actuated until time t₂. Time t₂ is the time that the user stops actuating the user control 14 (e.g. stops pressing switch 14, stops pulling trigger 14, stops pressing button 14, etc.). At time t₂ the first timer is started with duration dₜᵢₘₑᵣ₁, and thus the first timer expires at a time t₂ + dₜᵢₘₑᵣ₁. At some time after the start of the first timer and before the first timer expires at time t₂ + dₜᵢₘₑᵣ₁, a second timer is started, with the second timer having a duration such that it also expires at time t₂ + dₜᵢₘₑᵣ₁. The duration of the second timer is denoted dₜᵢₘₑᵣ₂, and thus the second timer is started at time t₂ + dₜᵢₘₑᵣ₁ - dₜᵢₘₑᵣ₂. In these embodiments, the trigger criterion is still satisfied between times t₁ and t₂ and while the first timer is running (i.e. not expired), and if the closure criterion is satisfied while the trigger criterion is satisfied, then a light pulse is generated, as in the above embodiment. However, if a light pulse has not been generated by the time that the second timer starts, the processing unit 32 can determine that a light pulse should be generated regardless of the closure criterion being satisfied. Therefore, if the closure criterion is not satisfied by time t₂ + dₜᵢₘₑᵣ₁ - dₜᵢₘₑᵣ₂, a light pulse can be generated in the time period 56 between (t2 + dₜᵢₘₑᵣ₁ - dₜᵢₘₑᵣ₂) and (t₂ + dₜᵢₘₑᵣ₁). The duration of the second timer may be of the order of 10 ms. Alternatively the duration of the second timer may be of the order of 20 ms. More generally, the duration of the second timer may be the duration of a typical software cycle of the processing unit 32 plus the typical duration of a light pulse. These embodiments (that use the second timer) have the benefit that the timing of the light pulse generation is matched to when the user blinks or otherwise sufficiently closes their eye(s) where possible, so that the user experience is improved, but ensures that a light pulse is generated in any case after the user actuates the user control 14. Without this 'override', a user may become frustrated if the user control 14 is actuated but no light pulse is generated.

It will be appreciated that the above embodiments with the trigger criterion, and with the trigger criterion with the override, can be combined with the earlier embodiments so that the criteria evaluated in step 105 can also include one or more of the contact criterion, the skin tone criterion and the charged criterion. In this case, the device 2 is activated to generate a light pulse in step 107 if all of these criteria are satisfied. However, if any one or more of these criteria is not satisfied, then the device 2 is not activated and no light pulse is generated, subject to the override in the latter embodiments, where the light pulse can be generated despite the closure criterion not being satisfied by the time that the second timer starts. However, it will be appreciated that in the override embodiments, the other criteria (i.e. contact criterion, the skin tone criterion and the charged criterion (as appropriate)) should be satisfied for the light pulse to be generated.

In a first specific embodiment, the criteria evaluated in step 105 comprises the closure criterion, the contact criterion, the skin tone criterion and the trigger criterion where the trigger criterion is satisfied only if, at the time that the trigger criterion is evaluated, a pulse trigger is being provided by the user. Therefore, a light pulse is generated if, at the same time, the eye(s) of the user are sufficiently closed, the device 2 is in contact with skin, the skin tone of the skin is below the skin tone threshold (or, in the alternative embodiments mentioned above: above the skin tone threshold or in the skin tone range), and a pulse trigger is being provided by the user. If there is no time where all of these criteria are satisfied, then a light pulse is not generated.

In a second specific embodiment, the criteria evaluated in step 105 comprises the closure criterion, the contact criterion, the skin tone criterion and the trigger criterion where the trigger criterion is satisfied if, at the time that the trigger criterion is evaluated, a pulse trigger is being provided by the user or has been provided by the user no more than a predetermined time ago. Therefore, a light pulse is generated if, at the same time, the eye(s) of the user are sufficiently closed, the device 2 is in contact with skin, the skin tone of the skin is below the skin tone threshold (or, in the alternative embodiments mentioned above: above the skin tone threshold or in the skin tone range), and either a pulse trigger is being provided by the user, or has been provided less than the first timer duration ago (e.g. less than 100 ms). If there is no time where all of these criteria are satisfied, then a light pulse is not generated.

In a third specific embodiment, the criteria evaluated in step 105 comprises the closure criterion, the contact criterion, the skin tone criterion, the override and the trigger criterion where the trigger criterion is satisfied if, at the time that the trigger criterion is evaluated, a pulse trigger is being provided by the user or has been provided by the user no more than a predetermined time ago. Therefore, a light pulse is generated either (1) if at the same time, the eye(s) of the user are sufficiently closed, the device 2 is in contact with skin, the skin tone of the skin is below the skin tone threshold (or, in the alternative embodiments mentioned above: above the skin tone threshold or in the skin tone range), and either a pulse trigger is being provided by the user, or has been provided less than the first timer duration ago (e.g. less than 100 ms) or (2) if at the same time, the device 2 is in contact with skin, the skin tone of the skin is below the skin tone threshold (or, in the alternative embodiments mentioned above: above the skin tone threshold or in the skin tone range), a pulse trigger has been provided less than the first timer duration ago (e.g. less than 100 ms) and the second timer is running (i.e. not expired). If there is no time where all of these criteria are satisfied, then a light pulse is not generated.

There is therefore provided techniques for the control of the generation of a light pulse by a device that can reduce the occurrence of after images in the vision of the user of the device, and improve the user's experience and/or eye safety in performing the treatment operation using the device.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An apparatus for controlling the generation of a light pulse by a device, wherein the device is configured to apply the light pulse to skin of a subject to perform a treatment operation on or to the skin, the apparatus comprising a processing unit configured to:
receive a first measurement signal from a first sensor configured to monitor at least one eye of a user of the device during use of the device;
process the first measurement signal to determine an amount of closure of the at least one eye of the user;
determine whether each of one or more criteria is satisfied, said one or more criteria comprising at least a closure criterion requiring the at least one eye of the user to be closed by at least a minimum amount of closure; and
activate the device to generate a light pulse if it is determined that each of said one or more criteria is satisfied.

2. An apparatus as claimed in claim 1, wherein the processing unit is configured to determine that the device is not to generate a light pulse if it is determined that any of the one or more criteria are not satisfied.

3. An apparatus as claimed in claim 1 or 2, wherein the one or more criteria further comprise a contact criterion requiring the device to be in contact with the skin.

4. An apparatus as claimed in any of claims 1-3, wherein the one or more criteria further comprise a skin tone criterion requiring a skin tone of the skin to be either: below a skin tone threshold, above a skin tone threshold, or in a skin tone threshold range.

5. An apparatus as claimed in any of claims 1-4, wherein the one or more criteria further comprise a charged criterion requiring a component in the device for causing a light source in the device to generate the light pulse to be charged.

6. An apparatus as claimed in any of claims 1-5, wherein the one or more criteria further comprise a trigger criterion requiring a pulse trigger to be provided by the user.

7. An apparatus as claimed in claim 6, wherein the one or more criteria further comprise a timer criterion requiring a first timer not to have expired, wherein the first timer is started after the pulse trigger is provided by the user.

8. An apparatus as claimed in claim 7, wherein the processing unit is further configured to:
start a second timer after the pulse trigger is provided by the user, wherein the second timer is set to expire at the same time as the first timer, and the second timer has a shorter duration than the first timer; and
activate the device to generate a light pulse if it is determined that a light pulse has not been generated since the pulse trigger was provided by the user and the second timer has been started.

9. A system for performing a treatment operation on or to skin of a subject, the system comprising:
a device for use by a user, wherein the device is for generating a light pulse that is to be applied to the skin of the subject to perform the treatment operation;
a first sensor for monitoring at least one eye of the user; and
an apparatus as claimed in any of claims 1-8 for controlling the generation of the light pulse by the device.

10. A method for controlling the generation of a light pulse by a device, wherein the device is configured to apply the light pulse to skin of a subject to perform a treatment operation on or to the skin, the method comprising:
receiving a first measurement signal from a first sensor configured to monitor at least one eye of a user of the device during use of the device;
processing the first measurement signal to determine an amount of closure of the at least one eye of the user;
determining whether each of one or more criteria is satisfied, said one or more criteria comprising at least a closure criterion requiring the at least one eye of the user to be closed by at least a minimum amount of closure; and
activating the device to generate a light pulse if it is determined that each of said one or more criteria is satisfied.

11. A method as claimed in claim 10, wherein the one or more criteria further comprise a contact criterion requiring the device to be in contact with the skin.

12. A method as claimed in claim 10 or 11, wherein the one or more criteria further comprise a skin tone criterion requiring a skin tone of the skin to be either: below a skin tone threshold, above a skin tone threshold, or in a skin tone threshold range.

13. A method as claimed in any of claims 10-12, wherein the one or more criteria further comprise a charged criterion requiring a component in the device for causing a light source in the device to generate the light pulse to be charged.

14. A method as claimed in any of claims 10-13, wherein the one or more criteria further comprise a trigger criterion requiring a pulse trigger to be provided by the user.

15. A computer program product comprising a computer readable medium having computer readable code embodied therein, the computer readable code being configured such that, on execution by a suitable computer or processor, the computer or processor is caused to perform the method of any of claims 10-14.
